(19) **Europäisches Patentamt / European Patent Office / Office européen des brevets**

(11) **EP 1 978 107 A1**

(12) # EUROPEAN PATENT APPLICATION

(43) Date of publication:
**08.10.2008 Bulletin 2008/41**

(51) Int Cl.:
*C12Q 1/68* (2006.01)

(21) Application number: **07108984.1**

(22) Date of filing: **25.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **03.04.2007 US 909826 P**

(71) Applicants:
- **CENTRE NATIONAL DE LA RECHERCHE SCIENTIFIQUE (CNRS)**
  **75016 Paris (FR)**
- **UNIVERSITE DE DROIT ET DE LA SANTE DE LILLE 2**
  **59800 Lille (FR)**
- **INSTITUT PASTEUR DE LILLE**
  **59000 Lille (FR)**

(72) Inventors:
- **Dina, Christian Rafaël**
  **75009 Paris (FR)**
- **Gallina Delamare, Sophie Catherine**
  **59260 Hellemmes (Lille) (FR)**
- **Chevre, Jean-Claude**
  **59000 Lille (FR)**
- **Meyre, David Jean-Claude**
  **59164 Marpent (FR)**
- **Froguel, Philippe**
  **93170 Bagnolet (FR)**

(74) Representative: **Warcoin, Jacques et al**
**Cabinet Régimbeau**
**20, rue de Chazelles**
**75847 Paris cedex 17 (FR)**

(54) **Fto gene polymorphisms associated to obesity and/or type II diabetes**

(57) The present invention provides means and methods for risk assessment and/or diagnosis and/or prognosis of obesity and/or type II diabetes in humans, based on the detection of nucleic acid biomarkers belonging to, or associated with, a set of SNPs in the *fatso* (*FTO*) gene.

The present invention also provides means and methods for identifying a SNP haplotype associated with obesity and/or type II diabetes susceptibility in humans, for selecting pharmaceutical agents useful in prevention and/or treatment of obesity and/or type II diabetes in humans, for haplotyping the *fatso* (*FTO*) gene in humans.

**EP 1 978 107 A1**

```
                                 SEQUENCE LISTING

<110> CENTRE NATIONAL DE LA RECHERCHE SCIENTIFIQUE (CNRS)

<120> FTO gene polymorphisms associated to obesity and/or
      type II diabetes

<130> D25418

<150> US 60/909 826
<151> 2007-04-03

<160> 2

<170> PatentIn version 3.3

<210> 1
<211> 20
<212> DNA
<213> Artificial sequence

<220>
<223> Fto primer

<400> 1
tgccatcctt gcctcgctca                                              20


<210> 2
<211> 20
<212> DNA
<213> Artificial sequence

<220>
<223> Fto primer

<400> 2
tgggggctga atggctcaca                                              20
```

**Description**

The present invention relates to means for diagnosing, prognosing, treating and/or preventing obesity and/or type II diabetes in humans.

More precisely, the present invention provides means and methods for risk assessment and/or diagnosis and/or prognosis of obesity and/or type II diabetes in humans, based on the detection of nucleic acid biomarkers belonging to, or associated with, a set of SNPs in the *fatso* (*FTO*) gene.

The present invention also provides means and methods for identifying a SNP haplotype associated with obesity and/or type II diabetes susceptibility in humans, for selecting pharmaceutical agents useful in prevention and/or treatment of obesity and/or type II diabetes in humans, for haplotyping the *fatso (FTO)* gene in humans.

Obesity is a condition in which the natural energy reserve, stored in the fatty tissue of humans and other mammals, is increased to a point where it is associated with certain health conditions or increased mortality.

Obesity is both an individual clinical condition and is increasingly viewed as a serious public health problem. Excessive body weight is now commonly known to predispose to various diseases, particularly cardiovascular diseases, sleep apnea, osteoarthritis, and diabetes (mellitus) type II. More precisely, obesity, especially central obesity (male-type or waist-predominant obesity), is an important risk factor for the "metabolic syndrome" ("syndrome X"), the clustering of a number of diseases and risk factors that heavily predispose for cardiovascular diseases. These are diabetes (mellitus) type II, high blood pressure, high blood cholesterol, and triglyceride levels (combined hyperlipidemia). An inflammatory state is present, which - together with the above - has been implicated in the high prevalence of atherosclerosis, and a prothrombotic state may further worsen cardiovascular risk.

In the clinical setting, obesity is typically evaluated by measuring BMI (for "body mass index"), waist circumference, and evaluating the presence of risk factors and comorbidities. In epidemiological studies, BMI alone is used as an indicator of prevalence and incidence of obesity. BMI is calculated by dividing the subject's weight in kilograms by the square of his/her height in metres:

$$BMI = (kg\,/\,m^2) \text{ or } BMI = [weight(lbs.) \times 703\,/\,height(inches)^2]$$

Generally, it is considered that:

- a BMI less than 18.5 is underweight
- a BMI of 18.5 - 24.9 is normal weight
- a BMI of 25.0 - 29.9 is overweight
- a BMI of 30.0 - 39.9 is obese
- a BMI of 40.0 or higher is severely (or morbidly) obese
- also, a BMI of 35.0 or higher in the presence of at least one other significant comorbidity is usually classified as morbid obesity.

Factors that have been suggested to contribute to the development of obesity include, not only overeating, but also:

- genetic factors and some genetic disorders (e.g., Prader-Willi syndrome);
- underlying illness (e.g., hypothyroidism);
- certain medications (e.g., atypical antipsychotics);
- sedentary lifestyle; etc.

Obesity is often given to result from a combination of genetic and non-genetic factors. In this respect, the causative gene(s) is(are) still to be identified.

Today, obesity is seen as the biggest health problem facing developed countries.

Among all the means that have been made available for combating obesity, bariatric surgery is being increasingly used. This technique consists of placing a silicone ring around the top of the stomach to help restrict the amount of food eaten in a sitting. Other more invasive surgery techniques, that cut into or reroute any of the digestive tract, have been also used. However, all of these surgeries comme with risk to the patient and they do not guarantee either successful weight loss or reduced morbidity and mortality.

As a consequence, there is a need in the art for new drugs that would be really efficient for combating obesity. In this regard, identifying the gene(s) that is(are) involved in obesity onset, and thus that is(are) promising candidate therapeutic target(s), is one of the more crucial concerns of scientists and medical staffs.

This is precisely this need that the present invention aims at satisfying by disclosing the most significant association reported so far between a genetic character and obesity. Indeed, the present invention is based on the finding that several SNPs (for "single nucleotide polymorphisms") in *fatso (FTO)* locus are highly and significantly associated with early onset and severe obesity, as well as with the obesity related diabetes (i.e., type II diabetes), in European population.

SNPs represent one of the most common forms of genetic variation. These polymorphisms appear when a single nucleotide in the genome is altered (such as via substitution, addition or deletion). Each version of the sequence with respect to the polymorphic site is referred to as an "allele" of the polymorphic site. SNPs tend to be evolutionary stable from generation to generation and, as such, can be used to study specific genetic abnormalities throughout a population. If SNPs occur in the protein coding region, it can lead to the expression of a variant, sometimes defective, form of the protein that may lead to development of a genetic disease. Some SNPs may occur in non-coding regions, but nevertheless, may result in differential or defective splicing, or altered protein expression levels. SNPs can therefore serve as effective indicators of a genetic disease. SNPs can also be used as diagnostic tools for identifying individuals with a predisposition for a disease, genotyping the individual suffering from the disease, and facilitating drug development based on the insight revealed regarding the role of target proteins in the pathogenesis process.

For the avoidance of doubt, the methods of the invention do not involve diagnosis practised on the human body. The methods of the invention are preferably conducted on a sample that has previously been removed from the individual. The kits of the invention, described hereunder, may include means for extracting the sample from the individual.

The methods of the invention allow the accurate evaluation of risk for an individual's health due to obesity and/or type II diabetes (e.g., risk of death, risk of complications, and the like) at or before disease onset, thus reducing or minimizing the negative effects of obesity and/or type II diabetes. The methods of the invention can be applied in persons who are free of clinical symptoms and signs of obesity and/or type II diabetes, in those who already have obesity and/or type II diabetes, in those who have family history of obesity and/or type II diabetes, or in those who have elevated level or levels of risk factors of obesity and/or type II diabetes.

In the context of the present invention, a "biomarker" (also herein referred to as a "marker") is a genetic marker indicative of obesity and/or type II diabetes in humans, that is to say a nucleic acid sequence which is specifically and significantly involved in obesity and/or type II diabetes onset. In the context of the invention, such a marker may also be called an "obesity and/or type II diabetes risk SNP marker" or a "risk SNP marker" or a "risk marker" or an "SNP marker".

Typically, the genetic markers used in the invention are particular alleles at "polymorphic sites" associated with obesity and/or type II diabetes. A nucleotide position in genome at which more than one sequence is possible in a population is referred to as a "polymorphic site". Where a polymorphic site is a single nucleotide in length, the site is commonly called an "SNP". For example, if at a particular chromosomal location, one member of a population has an adenine and another member of the population has a thymine at the same position, then this position is a polymorphic site and, more specifically, the polymorphic site is an SNP. Polymorphic sites may be several nucleotides in length due to, e.g., insertions, deletions, conversions, substitutions, duplications, or translocations. Each version of the sequence with respect to the polymorphic site is referred to as an "allele" of the polymorphic site. Thus, in the previous example, the SNP allows for both an adenine allele and a thymine allele. These alleles are "variant" alleles. Nucleotide sequence variants, either in coding or in non-coding regions, can result in changes in the sequence of the encoded polypeptide, thus affecting the properties thereof (altered activity, altered distribution, altered stability, etc.) Alternatively, nucleotide sequence variants, either in coding or in non-coding regions, can result in changes affecting transcription of a gene or translation of its mRNA. In all cases, the alterations may be qualitative or quantitative or both.

Those skilled in the art will readily recognize that the analysis of the nucleotides present in one or several of the SNP markers disclosed herein in an individual's nucleic acid can be done by any method or technique capable of determining nucleotides present in a polymorphic site. For instance, one may detect biomarkers in the methods of the present invention by performing sequencing, mini-sequencing, hybridisation, restriction fragment analysis, oligonucleotide ligation assay, allele-specific PCR, or a combination thereof. Of course, this list is merely illustrative and in no way limiting. Those skilled in the art may use any appropriate method to achieve such detection.

As it is obvious in the art, the nucleotides present in SNP markers can be determined from either nucleic acid strand or from both strands.

Importantly, the biomarkers used in the context of the invention are "associated with" the *FTO* gene, which means that said biomarkers are structurally associated with the *FTO* gene, e.g., the biomarkers are either in the *FTO* locus, or in close proximity thereto, and/or that said biomarkers are functionally associated with the *FTO* gene, e.g., the biomarkers interact with or affect the *FTO* gene or the expression product thereof.

Preferably, the biomarkers used in the methods and kits of the present invention are selected from the group of single nucleotide polymorphisms (SNPs) listed in Table 2 below (see paragraph II.1. in the Examples below). Yet preferably, the SNPs listed in Table 2 that are of highly significant predictive value are selected from rs9940128, rs1421085, rs1121980, rs17817449, rs3751812, rs11075990, and rs9941349. In this group, the SNPs rs9940128, rs1421085, rs1121980, and rs17817449 are of particular interest. Yet more preferably, one will use at least the SNP rs1421085 or rs17817449.

Alternatively, the biomarkers may be polymorphic sites associated with at least one SNP selected from the group listed in Table 2 below. As defined above, the terms "associated with" mean that said biomarkers are structurally and/or functionally associated with said SNP(s).

Yet alternatively, the biomarkers may be polymorphic sites being in complete linkage disequilibrium with at least one SNP selected from the group listed in Table 2 below.

Thus, a first aspect of the present invention concerns an *in vitro* method for risk assessment and/or diagnosis and/or prognosis of obesity and/or type II diabetes in a human subject, comprising at least:

a) detecting, in a nucleic acid sample from said human subject, at least one biomarker associated with the *FTO* gene; and

b) comparing the biomarker data obtained in step a) from said human subject to biomarker data from healthy and/or diseased people to make risk assessment and/or diagnosis and/or prognosis of obesity and/or type II diabetes in said human subject.

By "risk assessment", it is meant herein that the present invention makes it possible to estimate or evaluate the risk of a human subject to develop obesity and/or type II diabetes (one could also say "predisposition or susceptibility assessment"). In this respect, an individual "at risk" of obesity and/or type II diabetes is an individual who has at least one at-risk allele or haplotype with one or more "obesity and/or type II diabetes risk SNP markers". In addition, an "at-risk" individual may also have at least one risk factor known to contribute to the development of obesity and/or type II diabetes, including for instance:

- family history of obesity and/or type II diabetes;
- some genetic disorders, e.g., Prader-Willi syndrome;
- underlying illness (e.g., hypothyroidism);
- hypertension and elevated blood pressure;
- eating disorders;
- certain medications (e.g., atypical antipsychotics);
- sedentary lifestyle;
- a high glycemic diet, consisting of meals giving high postprandial blood sugar);
- weight cycling, caused by repeated attempts to lose weight by dieting;
- stressful mentality;
- insufficient sleep;
- smoking cessation, etc.

The prediction or risk generally implies that the risk is either increased or reduced.

There is no limitation on the type of nucleic acid sample that may be used in the context of the present invention. In this respect, one may used, e.g., a DNA sample, a genomic DNA sample, an RNA sample, a cDNA sample, an hnRNA sample, or an mRNA sample.

The "diseased" people referred to in the methods of the invention are people suffering from obesity and/or type II diabetes.

According to various embodiments, the method described above is useful for :

- identifying human subjects at risk for developing obesity and/or type II diabetes;
- diagnosing obesity and/or type II diabetes in a human subject;
- selecting efficient and safe therapy to a human subject having obesity and/or type II diabetes;
- monitoring the effect of a therapy administered to a human subject having obesity and/or type II diabetes;
- predicting the effectiveness of a therapy to treat obesity and/or type II diabetes in a human subject in need of such treatment;
- selecting efficient and safe preventive therapy to a human subject at risk for developing obesity and/or type II diabetes;
- monitoring the effect of a preventive therapy administered to a human subject at risk for developing obesity and/or type II diabetes;
- predicting the effectiveness of a therapy to prevent obesity and/or type II diabetes in a human subject at risk.

The terms "treatment" and "therapy" refer not only to ameliorating symptoms associated with obesity and/or type II diabetes, but also preventing or delaying the onset of the disease, and/or also lessening the severity or frequency of symptoms of the disease, and/or also preventing or delaying the occurrence of another episode of the disease.

A second aspect of the present invention relates to an *in vitro* method for identifying a SNP haplotype associated with obesity and/or type II diabetes susceptibility in a human subject, wherein said method comprises at least:

> a) detecting, in a nucleic acid sample from said human subject, at least one SNP of the *FTO* gene, wherein said at least one SNP is indicative of obesity and/or type II diabetes susceptibility; and
> b) identifying said SNP haplotype in said human subject, wherein said SNP haplotype comprises said at least one SNP detected in step a).

As it is well known in the art, a "haplotype" refers to any combination of genetic markers. A haplotype can comprise two or more alleles. The haplotypes (or "at-risk haplotypes") described herein are found more frequently and significantly in individuals at risk of obesity and/or type II diabetes than in individuals without obesity and/or type II diabetes risk. Therefore, these haplotypes have predictive value for detecting obesity and/or type II diabetes risk, or a susceptibility to obesity and/or type II diabetes in an individual. An "at-risk haplotype" is thus intended to embrace one or a combination of haplotypes described herein over the markers that show high and significant correlation to obesity and/or type II diabetes.

Detecting haplotypes can be accomplished by methods well known in the art for detecting sequences at polymorphic sites.

Preferably, the SNP(s) detected in step a) is(are) selected from the group listed in Table 2 below.

A third aspect of the present invention provides a test kit for using in an *in vitro* method to make risk assessment and/or diagnosis and/or prognosis of obesity and/or of type II diabetes in a human subject, wherein said test kit comprises appropriate means for:

> a) assessing type and/or level of at least one biomarker associated with the FTO gene in a nucleic acid sample from said human subject; and
> b) comparing the biomarker data assessed in a) from said human subject to biomarker data from healthy and/or diseased people to make risk assessment and/or diagnosis and/or prognosis of obesity and/or of type II diabetes in said human subject.

A fourth aspect of the present invention is related to a test kit for using in an *in vitro* method for identifying a SNP haplotype associated with obesity and/or type II diabetes susceptibility in a human subject, comprising appropriate means for:

> a) detecting at least one SNP of the FTO gene in a nucleic acid sample from said human subject, wherein said at least one SNP is indicative of obesity and/or type II diabetes susceptibility; and
> b) identifying SNP haplotype in said human subject, wherein said SNP haplotype comprises said at least one SNP detected in a).

The terms "test kit" and "kit" are synonymous and may be used interchangeably.

In the context of the present invention when reference is made to test kits, the terms « appropriate means » refer to any technical means useful for achieving the indicated purpose. As non-limiting examples of such appropriate means, one can cite reagents and/or materials and/or protocols and/or instructions and/or software, etc. All the kits of the present invention may comprise appropriate packaging and instructions for use in the methods herein disclosed. The kits may further comprise appropriate buffer(s) and polymerase(s) such as thermostable polymerases, for example Taq polymerase. Such kits may also comprise control primers and/or probes.

According to preferred embodiments, the test kits of the invention may comprise at least:

> a) one isolated PCR primer pair consisting of a forward primer and a reverse primer, for specifically amplifying nucleic acids of interest; and/or
> b) one isolated primer for specifically extending nucleic acids of interest; and/or
> c) one isolated nucleic acid probe specifically binding to nucleic acids of interest; and/or
> d) one isolated antibody specifically binding protein() encoded by nucleic acid(s) of interest; and/or
> e) one microarray or multiwell plate comprising at least one of a) to d) above.

By "nucleic acids of interest", it is meant herein the nucleic acid regions or segments containing the biomarkers that are indicative of obesity and/or type II diabetes. In this respect, the nucleic acids of interest may be larger than the biomarkers or they may be limited to the biomarkers.

"Probes" and "primers" are oligonucleotides that hybridize in a base-specific manner to a complementary strand of nucleic acid molecules. By "base-specific manner", it is meant that the two sequences must have a degree of nucleotide complementarity sufficient for the primer or the probe to hybridize. Accordingly, the primer or probe sequence is not required to be perfectly complementary to the sequence of the template. Non-complementary bases or modified bases can be interspersed into the primer or probe, provided that base substitutions do not inhibit hybridization.

A probe or primer usually comprises a region of nucleic acid that hybridizes to at least about 8, preferably about 10, 12, 15, more preferably about 20, 25, 30, 35, and in some cases, about 40, 50, 60, 70 consecutive nucleotides of the nucleic acid template.

The primers and probes are tyupically at least 70% identical to the contiguous or complementary nucleic acid sequence (which is the "template"). Identity is preferably of at least 80%, 90%, 95%, and more preferably, of 98%, 99%, 99.5%, 99.8%.

Advantageously, the primers and probes further comprise a label, e.g., radioisotope, fluorescent compound, enzyme, or enzyme co-factor.

A fifth aspect of the present invention is directed to a method for selecting pharmaceutical agents useful in prevention and/or treatment of obesity and/or type II diabetes in a human subject, comprising at least:

a) administering the candidate agents to a model living system containing the human *FTO* gene;

b) determining the effect of said candidate agents on biological mechanisms involving said *FTO* gene and/or the expression product thereof; and

c) selecting the agents having an altering effect on said biological mechanisms, wherein the selected agents are considered useful in prevention and/or treatment of obesity and/or type II diabetes in a human subject.

By "pharmaceutical agent", it is referred to either biological agents or chemical agents or both, provided they can be considered as useful in prevention and/or treatment of obesity and/or type II diabetes in a human subject. Examples of biological agents are nucleic acids, including siRNAs; polypeptides, including toxins, enzymes, antibodies, either polyclonal antibodies or monoclonal antibodies; combinations of nucleic acids and polypeptides, and the like. Examples of chemical agents are chemical molecules, chemical molecular complexes, chemical moieties, and the like (e.g., radioisotopes, etc.).

In a sixth aspect, the present invention concerns the use of a model living system containing the human *FTO* gene for studying pathophysiology and/or molecular mechanisms involved in obesity and/or type II diabetes.

Where reference is made herein to a "model living system", it is preferably referred to a non-human transgenic animal, or a cultured microbial, insect or mammalian cell, or a mammalian tissue or organ. More preferably, said model living system will express or overexpress the human *FTO* gene.

A seventh aspect of the present invention relates to an *in vitro* method for haplotyping the *FTO* gene in a human subject, comprising at least:

a) detecting, in a nucleic acid sample from said human subject, the nucleotides present at each allelic position of an "obesity and/or type II diabetes susceptibility haplotype", which haplotype includes at least one of the SNPs listed in Table 2 or a polymorphism in linkage disequilibrium therewith; and

b) assigning said human subject a particular haplotype according to the nucleotides detected in a).

In a preferred embodiment, this method further comprises the step of determining the risk of said human subject for developing obesity and/or type II diabetes according to the particular haplotype assigned in step b).

The nucleotides present at each allelic position may be detected in step a) of the above method using any appropriate techniques. For instance, this detection may be performed using enzymatic amplification, such as polymerase chain reaction or allele-specific amplification, of said nucleic acid sample. Alternativeley, said detection may be done using sequencing.

Besides, the SNPs and haplotypes disclosed herein allow patient stratification. The subgroups of individuals identified as having increased or decreased risk of developing obesity and/or type II diabetes can be used, inter alia, for targeted clinical trial programs and pharmacogenetic therapies wherein knowledge of polymorphisms is used to help identify patients most suited to therapy with particular pharmaceutical agents.

The SNPs and haplotypes described herein represent a valuable information source helping to characterise individuals in terms of, for example, their identity and susceptibility to disease onset/development or susceptibility to treatment with particular drugs.

Therefore, an eighth aspect of the present invention is directed to a method for selecting human subjects for participation in a clinical trial to assess the efficacy of a therapy for treating and/or preventing obesity and/or type II diabetes, comprising at least:

a) grouping the human subjects according to the particular *FTO* gene haplotype that each human subject belongs to; and

b) selecting at least one human subject from at least one haplotype groups obtained in a) for inclusion in said clinical trial.

In this method, the particular *FTO* gene haplotype is advantageously determined *in vitro* by detecting, in a nucleic acid sample from each human subject, the nucleotides present at each allelic position of an "obesity and/or type II diabetes susceptibility haplotype", which haplotype includes at least one of the SNPs listed in Table 2 or a polymorphism in linkage disequilibrium therewith.

A ninth aspect of the present invention provides a test kit for *in vitro* haplotyping the FTO gene in a human subject according to the method as described above, wherein said test kit comprises appropriate means for::

a) detecting, in a nucleic acid sample from said human subject, the nucleotides present at each allelic position of an "obesity and/or type II diabetes susceptibility haplotype", which haplotype includes at least one SNP selected from the group listed in Table 2 or a polymorphism in linkage disequilibrium therewith; and
b) assigning said human subject a particular haplotype according to the nucleotides detected in a).

In addition, the present invention concerns, in a tenth aspect, the use of a test kit as described above for stratifying human subjects into particular haplotype groups.

Advantageously, this test kit is further used for selecting at least one human subject from at least one haplotype groups for inclusion in a clinical trial to assess the efficacy of a therapy for treating and/or preventing obesity and/or type II diabetes.

In an eleventh aspect, the present invention is related to a test kit for *in vitro* determining the identity of at least one SNP selected from the group listed in Table 2 in the human *FTO* gene, comprising appropriate means for such determination.

The present invention is illustrated by the non-limiting following figures:

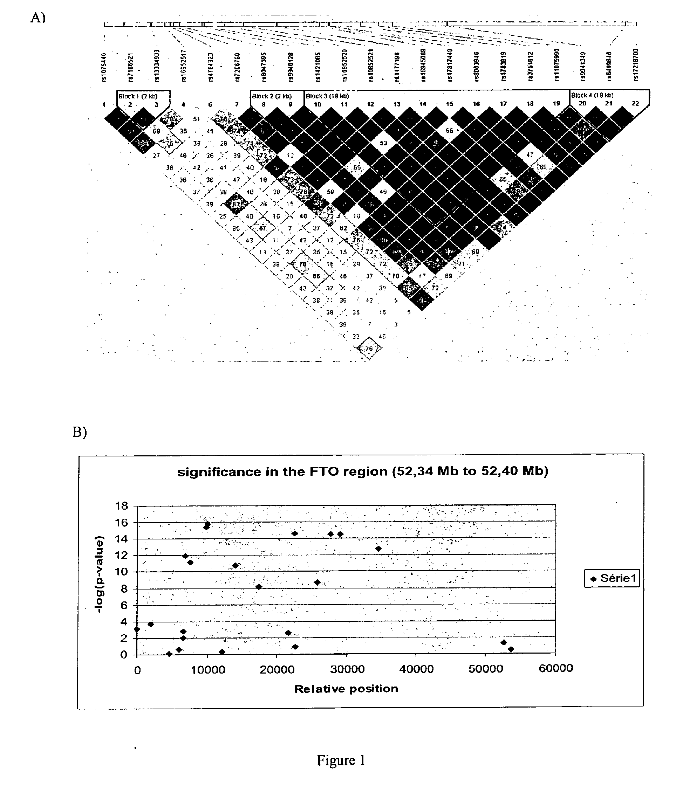

Figure 1: Linkage disequilibrium structure and association in the *FTO* region.

A) The linkage disequilibrium is presented as a 2 by 2 matrix where dark grey represents very high linkage disequilibrium ($r^2$) and white absence of correlation between SNPs.
b) For each of the SNPs, the $\log_{10}$ of the p-value for the class III obesity (880 individuals) vs. controls (2700) analysis is shown.

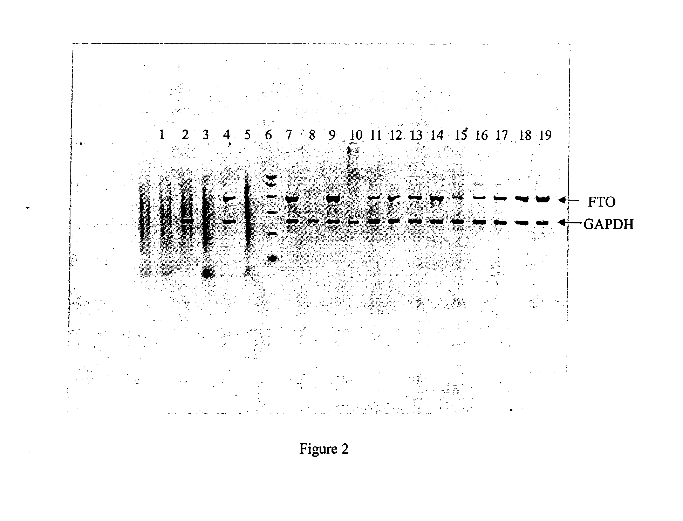

Figure 2: *FTO* gene expression in human tissues.
FTO expression in human cDNA from adipose tissue (BioChain Institute, USA), pancreatic islets, FACS-purified beta cells (provided by the Human Pancreatic Cell Core Facility, University Hospital, Lille, France) and multiple tissue cDNA panel (BD Biosciences Clontech) where 1 : FTO negative control, 2 : GAPDH, 3: GAPDH negative control, 4 : GAPDH+FTO, 5 : GAPDH+FTO negative control, 6 : molecular weight markers 50bp, 150bp, 300bp, 500bp, 750bp and 1 kb, 7 : adipose tissue, 8 : adipose tissue RT minus control, 9 : pancreatic islets, 10 : pancreatic islets RT minus control, 11 : heart, 12 : brain, 13 : placenta, 14 : lung, 15 : liver, 16 : skeletal muscle, 17 : kidney, 18 : pancreas, 19 : pancreatic beta cells. Glyceraldehyde-3-phosphate dehydrogenase (GAPDH) was used as internal control. Beta cell purity was confirmed by immunochemistry (98% insulin-positive cells) and PCR (absence of amplification with chymotrypsin primers, specific for exocrine cells, and presence of amplification with Pdx1 primers, specific for beta cells). FTO primers used were 5'-TGCCATCCTTGCCTCGCTCA-3' (SEQ ID No.1) and 5'-TGGGGGCTGAATGGCTCACA-3' (SEQ ID No.2). These two primers were high-performance liquid chromatography purified. 1$\mu$g of adipose tissue, pancreatic islets and beta cells RNA was randomly reverse transcribed using M-MLV Reverse Transcriptase (Promega, USA) according to instructions. PCR was performed using the FastStart Taq DNA polymerase kit (Roche, Germany) according to instructions with 1.25 mmol/l MgCl$_2$, 0.4 $\mu$mol/l of each primer, and 5 $\mu$l single strand cDNA, using the hot-start PCR method modified as follows: 95°C for 4 min, 40 cycles of 95°C for 30 s, 68°C for 2 min, and then 68°C for 3 min. PCR products were separated on 2% (wt/vol) agarose gel and visualized using ethidium bromide and ultraviolet trans-illumination.

Other embodiments and advantages of the present invention will be understood upon reading the following Examples.

**EXAMPLES**

**I. Materials and Methods:**

**1.1: Statistical analyses**

a) *Association tests.* Logistic regression was used to test association in case-controls under a multiplicative model and Pearson chi-square for the general association model.

The p-values for replication are one-sided for testing the specific hypothesis of increased frequency of allele C (resp. G) in SNPs rs1421085 (resp. rs17817449) in obese children and adults.

Association testing of both SNPs in family based cohorts was performed using the TDT test which compares the number of transmissions of the at-risk allele, from heterozyguous parent to affected offspring, to its expectation. A McNemar $\chi^2$ test assesses the significance.

Fisher's method was used for combining p-values of the different studies, in which the twice the negative sum of the natural log of $n$ p-values follows a $\chi^2$ distribution with $2n$ degrees of freedom.

b) *Genetic model.* The proportion of BMI variance explained in adult founders of our familial study populations (parents of French obese children) and in children from the Leipzig cohort, was estimated. The BMI was normalized and expressed in SDS.

The QTL liability threshold model with a quantitative liability trait L (mean 0 and SD 1 in the whole population) and a threshold T, above which an individual is classified as affected, was used. The trait L follows a mixture of three normal distributions $N(\mu_g, \sigma_R)$. $\mu_g$ is the genotype specific L mean (takes values -a, 0 and a) and $\sigma^2_R$ is the proportion of residual variance which is not due to the locus. For obesity, the trait L can be identified with BMI, as obesity is defined as having a BMI over a certain threshold. With these parameters, it was possible to express the disease risk in terms of Genotype Risk Ratios, $GRR_i = P(affected/G=i)/P(affected/G=0)$. The variance due to the locus under investigation was directly derived from the values a and *f*, the frequency of the at-risk allele in population : $\sigma^2_\alpha = 2.f.(1-f).a^2$ (Eq 1). The percentage of variance explained by the variant ($\sigma^2_\alpha$) was derived from the linear regression model, by inverting Eq 1. Then, the GRRs corresponding to a prevalence of 10%, used for common obesity, was iteratively calculated.

**I.2: Genotyping**

Initial case-control genotyping was done by the Applied Biosystems SNPlex™ Technology based on the Oligonucleotide Ligation Assay (OLA) combined with multiplex PCR target amplification (http:,'/www.appliedbiosystems.com). The chemistry of the assay relies on a set of universal core reagent kits and a set of SNP-specific ligation probes allowing a multiplex genotyping of 48 SNPs simultaneously in a unique sample. A quality control measure was included by using specific internal controls for each step of the assay (according to the manufacturer's instructions). Allelic discrimination was performed through capillary electrophoresis analysis using an Applied Biosystems 3730xl DNA Analyzer and GeneMapper3.7 software. Duplicate samples were assayed with a concordance rate of 100%.

High-throughput genotyping for the variants rs1421085 and rs17817449 in replication samples was performed using the TaqMan® SNP Genotyping Assays (Applied Biosystems, Foster City, Calif. USA). The PCR primers and TaqMan probes were designed by Primer Express and optimized according to the manufacturer's protocol.

All SNPs were in Hardy-Weinberg equilibrium (p>0.05). The call rates were higher than 95% in all and groups of cases and controls from all populations except in Swiss obese individuals.

Call rates and HWE test p-values are displayed in Table 1 below.

**Table 1** Genotype counts, Hardy Weinberg tests and percentage of successful genotyping

| | Cases | | | | | Controls | | | | |
|---|---|---|---|---|---|---|---|---|---|---|
| Study | CC(GG) | CT(GT) | TT | PHWE | Missing | CC(GG) | CT(GT) | TT | PHWE | Missing |
| French population, adult obesity | | | | | | | | | | |
| | 473 | 1273 | 944 | P=0.47 | 2.7% | 242 | 425 | 200 | p=0.88 | 3.2% |
| | 439 | 1288 | 948 | P=0.99 | 3.2% | 235 | 426 | 212 | P=0.79 | 2.5% |

(continued)

| French population, childhood obesity, study 1 | | | | | | | | | |
|---|---|---|---|---|---|---|---|---|---|
| 175 | 481 | 323 | P=0.98 | 3% | 192 | 334 | 173 | p=0.52 | 4.6% |
| 164 | 489 | 337 | P=0.84 | 2% | 205 | 320 | 164 | p=0.2 | 5.6% |
| French population, childhood obesity, study 2 | | | | | | | | | |
| 95 | 237 | 187 | P=0.43 | 2.5% | 130 | 233 | 19 | p=0.77 | 4.7% |
| 92 | 237 | 196 | P=0.38 | 1.3% | 129 | 236 | 120 | p=0.84 | 4.1 % |
| Swiss population, adult obesity | | | | | | | | | |
| 120 | 233 | 161 | P=0.14 | 5% | 146 | 235 | 123 | p=0.34 | 9% |
| 109 | 246 | 164 | P=0.64 | 4% | 135 | 243 | 138 | p=0.41 | 7% |
| German population, childhood obesity | | | | | | | | | |
| 110 | 343 | 246 | P=0.87 | 1.7% | 79 | 142 | 62 | p=0.99 | 0.9% |
| 119 | 341 | 231 | P=0.84 | 2.9% | 81 | 142 | 58 | p=0.96 | 1.4% |

pHWE: p-value for Hardy Weinberg disequilibrium test.

Missing: percentage of failed genotypes.

Genotype counts for CC, CT and TT (SNP rs1421085), GG, GT and TT (rs17817449) are presented in Table 1 above.

An unusually high frequency of C (resp. G) allele was observed in controls of the Swiss study which is the control sample with highest missing genotypes rate. This may be due either to presence of undetected obese individuals in this anonymous donors sample or be indicative of a correlation between call rate and allele frequency (differential call rate). However, the samples with the highest call rate and displaying no difference of missing rate between cases and controls (French adult obesity and German children obesity) showed the usual range of allele frequency difference (0.41 to 0.51). Thus, the observed association is unlikely to be due to genotype-dependent calling rate difference in cases and controls.

Besides usual duplicates, 535 obese children and 329 class III obese adults were genotyped both in the case-control and in the familial studies. The concordance rates between these two genotyping techniques were 100% for both SNPs in both studies.

## II. <u>Results:</u>

### II.1: Results of obesity studies

48 SNPs in different intergenic regions were initially selected in order to estimate the distribution of neutral SNPs in French Caucasian case-control obesity data-sets. Surprisingly, SNP rs1121980, located on chromosome 16q12.2, was found to be strongly associated with severe class III (BMI > 40kg/m$^2$) adult obesity (OR=1.55 [1.39-1.73], p-value=5.3.10$^{-16}$).

It appeared that this SNP is actually located within the first intron of a newly described gene named *fatso* or *FTO* (Peters *et al.,* 1999) that has nine predicted exons in humans and encompasses a large 410,507 bp. genomic region on the NCBI 36.1 human genome assembly, which is posterior to the SNPs selection. Additional SNPs were tested in a 60-kb region (30 kb on each side of this SNP) which spans the block where rs1121980 lies. This region encompasses part of the first intron, second exon and first part of the second intron of the *FTO* gene. SNPs tagging all the frequent markers (MAF > 0.05) with an $r^2$ > 0.7 as well as SNPs located in potentially functional elements (transcription factor binding sites or other regulatory elements and conserved region between species) and in $r^2$ > 0.8 with the initial SNP rs1121980, were selected. Twenty-five SNPs were eventually selected, and twenty-three were successfully genotyped. The case control sample comprised 896 class III obese adults (BMI >40kg/m$^2$), and 2,700 non obese French Caucasian controls (BMI < 27kg/m$^2$). Both obese adult individuals and controls have been previously described (Meyre *et al.,* 2005).

Results are shown in figure 1. Strong association of several SNPs with class III obesity (1.9.10$^{-16}$ < p < 5.10$^{-9}$) was found. Interestingly, three out of the five most significantly associated SNPs, rs17817449, rs3751812 and rs1421085 were putatively functional, based both on phastCons conservation score calculated on 11 vertebrates species (Siepel *et al.,* 2005) and Regulatory Potential score calculated on 7 species (King *et al.,* 2005). Information for all the genotyped SNPs is displayed in Tables 2 and 3 below.

**Table 2**

| | Status | MAF | $N_{11}$ (%) | $N_{12}$ (%) | $N_{22}$ (%) | general | additive |
|---|---|---|---|---|---|---|---|
| | | | | | | | |
| | | | Genotype distribution and association tests under the general and the additive model | | | | |
| rs1075440 | Non Obese | 0,312 | 1137 (0.47) | 1026 (0.43) | 236 (0.10) | | |
| | Class III | 0,269 | 473 (0.54) | 330 (0.38) | 70 (0.08) | $2,398.10^{-03}$ | $7,877.10^{-04}$ |
| rs7186521 | Non Obese | 0,468 | 672 (0.28) | 1203 (0.50) | 519 (0.22) | | |
| | Class III | 0,521 | 203 (0.23) | 423 (0.49) | 239 (0.28) | $5,761 .10^{-04}$ | $1,670.10^{-04}$ |
| rs13334933 | Non Obese | 0,188 | 1584 (0.66) | 734 (0.31) | 85 (0.04) | | |
| | Class III | 0,184 | 582 (0.66) | 266 (0.30) | 28 (0.03) | $8,827.10^{-01}$ | $6,892.10^{-01}$ |
| rs16952517 | Non Obese | 0,118 | 1868 (0.78) | 506 (0.21) | 31 (0.01) | | |
| | Class III | 0,126 | 673 (0.77) | 188 (0.21) | 17 (0.02) | $3,731.10^{-01}$ | $3,594.10^{-01}$ |
| rs6499643 | Non Obese | 0,159 | 1625 (0.72) | 573 (0.25) | 74 (0.03) | | |
| | Class III | 0,130 | 639 (0.77) | 175 (0.21) | 21 (0.03) | $2,01 9.10^{-02}$ | $5,741 .10^{-03}$ |
| rs4784323 | Non Obese | 0,326 | 1080 (0.45) | 1080 (0.45) | 243 (0.10) | | |
| | Class III | 0,283 | 449 (0.51) | 361 (0.41) | 68 (0.08) | $3,618.10^{-03}$ | $7,793.10^{-04}$ |
| rs7206790 | Non Obese | 0,524 | 560 (0.23) | 1176 (0.49) | 675 (0.28) | | |
| | Class III | 0,423 | 292 (0.34) | 407 (0.47) | 160 (0.19) | $1,251.10^{-11}$ | $1,439.10^{-12}$ |
| rs8047395 | Non Obese | 0,481 | 655 (0.27) | 1187 (0.49) | 562 (0.23) | | |
| | Class III | 0,383 | 332 (0.38) | 413 (0.47) | 128 (0.15) | $3,104.10^{-11}$ | $2,626.10^{-12}$ |
| rs9940128 | Non Obese | 0,426 | 811 (0.34) | 1155 (0.48) | 453 (0.19) | | |
| | Class III | 0,537 | 190 (0.22) | 420 (0.49) | 254 (0.29) | $4,317.10^{-14}$ | $4,706.10^{-15}$ |
| rs1421085 | Non Obese | 0,410 | 855 (0.35) | 1134 (0.47) | 422 (0.18) | | |
| | Class III | 0,524 | 200 (0.23) | 425 (0.49) | 242 (0.28) | $7,392.10^{-15}$ | $7,605.10^{-16}$ |
| rs16952520 | Non Obese | 0,042 | 2201 (0.92) | 200 (0.08) | 2 (0.00) | | |
| | Class III | 0,038 | 816 (0.93) | 65 (0.07) | 1 (0.00) | $6,535.10^{-01}$ | $4,131.10^{-01}$ |
| rs10852521 | Non Obese | 0,479 | 673 (0.28) | 1171 (0.48) | 572 (0.24) | | |
| | Class III | 0,386 | 327 (0.38) | 415 (0.48) | 129 (0.15) | $3,713.10^{-10}$ | $3,712.10^{-11}$ |
| rs1477196 | Non Obese | 0,368 | 967 (0.40) | 1112 (0.46) | 329 (0.14) | | |
| | Class III | 0,290 | 438 (0.51) | 340 (0.40) | 78 (0.09) | $3,752.10^{-08}$ | $5,922.10^{-09}$ |
| rs1121980 | Non Obese | 0,429 | 892 (0.33) | 1270 (0.48) | 511 (0.19) | | |
| | Class III | 0,541 | 189 (0.21) | 436 (0.49) | 262 (0.30) | $5,697.10^{-15}$ | $5,277.10^{-16}$ |
| rs16945088 | Non Obese | 0,089 | 2001 (0.83) | 394 (0.16) | 17 (0.01) | | |
| | Class III | 0,067 | 750 (0.87) | 109 (0.13) | 3 (0.00) | $1,666.10^{-02}$ | $3,493.10^{-03}$ |
| rs17817449 | Non Obese | 0,402 | 860 (0.36) | 1152 (0.48) | 388 (0.16) | | |
| | Class III | 0,513 | 212 (0.24) | 426 (0.49) | 235 (0.27) | $7,554.10^{-15}$ | $1,442.10^{-15}$ |
| rs8063946 | Non Obese | 0,057 | 2150 (0.89) | 258 (0.11) | 8 (0.00) | | |
| | Class III | 0,048 | 782 (0.91) | 80 (0.09) | 1 (0.00) | $2,886.10^{-01}$ | $1,416.10^{-01}$ |
| rs4783819 | Non Obese | 0,371 | 943 (0.39) | 1122 (0.47) | 325 (0.14) | | |
| | Class III | 0,289 | 443 (0.52) | 337 (0.39) | 80 (0.09) | $2,780.10^{-09}$ | $8,596.10^{-10}$ |

(continued)

| | Status | MAF | N$_{11}$ (%) | N$_{12}$ (%) | N$_{22}$ (%) | general | additive |
|---|---|---|---|---|---|---|---|
| | | | | | | | |
| | Genotype distribution and association tests under the general and the additive model | | | | | | |
| rs3751812 | Non Obese | 0,399 | 885 (0.37) | 1141 (0.47) | 394 (0.16) | | |
| | Class III | 0,505 | 218 (0.25) | 425 (0.49) | 226 (0.26) | 3,038.10$^{-13}$ | 4,121 .10$^{-14}$ |
| rs11075990 | Non Obese | 0,401 | 871 (0.36) | 1146 (0.48) | 394 (0.16) | | |
| | Class III | 0,509 | 211 (0.25) | 421 (0.49) | 227 (0.26) | 1,037.10$^{-13}$ | 1,483.1 0$^{-14}$ |
| rs9941349 | Non Obese | 0,412 | 843 (0.35) | 1154 (0.48) | 417 (0.17) | | |
| | Class III | 0,513 | 211 (0.24) | 422 (0.49) | 233 (0.27) | 4,986.10$^{-12}$ | 7,420.10$^{-13}$ |
| rs6499646 | Non Obese | 0,096 | 1965 (0.81) | 434 (0.18) | 16 (0.01) | | |
| | Class III | 0,079 | 747 (0.85) | 123 (0.14) | 8 (0.01) | 2,238.10$^{-02}$ | 2,797.10$^{-02}$ |
| rs17218700 | Non Obese | 0,120 | 1859 (0.77) | 518 (0.22) | 31 (0.01) | | |
| | Class III | 0,111 | 688 (0.80) | 159 (0.18) | 16 (0.02) | 8,785.10$^{-02}$ | 2,774.10$^{-01}$ |

MAF: the minor allele frequency.

N$_{11}$, N$_{12}$ and N$_{22}$ are the genotype frequencies for the frequent allele homozygote, the heterozygote and the rare allele homozygote, respectively.

General: result of the general test model test, a Pearson $\chi^2$ test with 2 degrees of freedom comparing the genotype frequencies in case and control.

Additive: result of the logistic regression of the case-control status on the number of at-risk alleles.

**Table 3**

| Assessment of SNP's putative functionality | | | | |
|---|---|---|---|---|
| position | rs | genomatix | conservation | Regulatory Potential |
| 52357007 | rs9937053 | | 0 | - |
| 52357405 | rs9928094 | | 0.000708661 | 0 |
| 52357477 | rs9930333 | | 0.00283465 | 0 |
| 52358068 | rs9939973 | | 0.00283465 | 0 |
| 52358129 | rs9940646 | | 0 | 0.0695239 |
| 52358254 | rs9940128 | | 0 | 0.24681 |
| **52358454** | **rs1421085** | | 1 | 0.31981 |
| 52359049 | rs9923147 | | 0.0136693 | 0.0697798 |
| 52359485 | rs9923544 | | 0 | 0 |
| 52361074 | rs1558902 | | 0 | 0.0609172 |
| 52362707 | rs11075985 | | 0 | 0 |
| 52366747 | rs1121980 | | 0 | 0 |
| 52368186 | rs7193144 | | 0 | 0 |
| **52370867** | **rs17817449** | * | 0.992126 | 0.286477 |
| 52370950 | rs8043757 | | 0.0393701 | 0.0860575 |
| 52373775 | rs8050136 | | 0.304016 | 0.198246 |
| 52374252 | rs8051591 | | 0 | 0 |
| 52374338 | rs9935401 | | 0.00179528 | 0 |

(continued)

| Assessment of SNP's putative functionality | | | | |
|---|---|---|---|---|
| position | rs | genomatix | conservation | Regulatory Potential |
| **52375960** | **rs3751812** | * | 1 | 0.326163 |
| 52376669 | rs9936385 | | 0.0393701 | 0.029236 |
| 52376698 | rs9923233 | | 0 | 0.0498732 |
| 52377377 | rs11075989 | | 0 | - |
| 52377393 | rs11075990 | | 0 | - |
| 52379362 | rs7201850 | | 0.0060315 | 0 |
| 52380151 | rs7185735 | | 0 | 0 |
| 52382988 | rs9941349 | | 0.76378 | 0 |
| 52384679 | rs9931494 | | 0 | 0.00920513 |
| 52385566 | rs17817964 | | 0.661748 | 0.158021 |
| 52387952 | rs9930501 | | 0 | 0 |
| 52387965 | rs9930506 | | 0 | 0 |
| 52387991 | rs9932754 | | 0.0530236 | 0 |

For each SNP, it is reported in Table 3 above the physical position in bp using NCBI assembly Build 35, the phastCons conservation score calculated on 11 vertebrates species and Regulatory Potential score calculated on 7 species. A star is added when the SNP inserts or deletes a Transcription Factor Binding Site (using SNP inspector Tool from Genomatix Suite). In bold are indicated the three SNPs having the highest scores and then being most likely functional.

It was also tested whether the association observed in the whole region was reflecting one unique signal or whether any other SNP or haplotype displays association on its own, and concluded that the at-risk alleles were nearly perfect proxies of each other. Thus, at least these three SNPs are likely to mirror one unique association of a haplotype combining derived alleles (from NCBI) with a frequency of 40% in controls.

As recently outlined (Ott, 2004), the replication of association data in additional samples is necessary to exclude spurious conclusions, especially when the pre study odd for the implication of a gene is low, which is the case for *fatso.* SNPs rs1421085 and rs17817449 were chosen, because they display very high evidence of association and are putatively functional, to carry out these analyses. All the p-values were one-sided in these analyses.

It was first compared allele frequencies of the selected SNPs in 1,010 non obese French individuals (Hercberg *et al.* 1998) (SUVIMAX cohort, BMI < 27 kg/m[2]) with 736 obese children (mean age = 11 y, BMI > 97th percentile) and found significant association with early onset obesity (1.28 [1.11 - 1.47] p=2.10$^{-5}$ and OR=1.25 [1.09 - 1.44] p=5.10$^{-4}$ respectively). Then, 532 non obese young French adults (Vu-Hong *et al.,* 2006) (Haguenau cohort, median age=21y, BMI < 25 kg/m[2]) and 505 French obese children with a BMI > 97th percentile (Le Fur *et al.,* 2002) from Saint Vincent de Paul Hospital, were analyzed. Again, similar trend for association with early onset obesity was found (OR=1.47 [1.23-1.75], p=1.17.10$^{-5}$ and OR=1.52 [1.28-1.81], p=1.82.10$^{-6}$, respectively). Finally, 700 lean children (mean age = 11.7y, BMI between 16th and 85th percentile) and 283 obese children (mean age = 11.7y, BMI >90th percentile), both of German Caucasian origin (Korner *et al.,* 2007), were genotyped. Association was again confirmed for both SNPs (1.69 [1.38 - 2.06], p=3.46.1 0-7 , and 1.65 [1.35 - 2.01], p=1.23.10$^{-6}$). Table 4 below shows the effect size estimation.

**Table 4**

| Effect size estimation | | | | | | |
|---|---|---|---|---|---|---|
| | m ZBMI | m Age | a | $\sigma^2_a$ | GRR$_1$ | PAR |
| French | 1.02 SDS | 55 y | 0.19 [0.09-0.28] | 0.017 [0.004-0.038] | 1.41 [1.17-1.69] | 0.27 [0.13-0.40] |
| German | 0.46 SDS | 11.7 y | 0.12 [0.03-0.20] | 0.007 [0.0005-0.019] | 1.24 [1.05-1.44] | 0.18 [0.04-0.29] |

(continued)

| Effect size estimation | | | | | | |
|---|---|---|---|---|---|---|
| | m ZBMI | m Age | a | $\sigma^2_a$ | GRR$_1$ | PAR |
| | | | | | 1.31 [1.16-1.48] | 0.22 [0.12-0.31] |
| m ZBMI: mean of the BMI expressed in standard deviations<br>m Age: mean age in the study population<br>a: additive effect, estimated by the slope of the regression of ZBMI on the number of at-risk alleles<br>$\sigma^2_a$: genetic variance (it is here assumed no deviation from the additive model). As the whole variance is 1, the genetic variance is equivalent to heritability<br>GRR$_1$: Genotype Risk Ratio between penetrance of wild type homozygote and heterozygote<br>PAR: population attributable risk | | | | | | |

557 Swiss class III obese adults and 541 anonymous Swiss donors were also genotyped, and it was further replicated the initial association between *fatso* and obesity (OR=1.26 [1.07-1.49], p=0.0032 and OR=1.21 [1.02-1.43] p=0.01). Of note, although allele frequencies in Swiss obese subjects were consistent with the initial observations in French obese subjects (MAF=0.50), the Swiss blood donor cohort which was not tested for obesity displayed higher allele frequencies (f=0.46 vs. 0.41), which may be explained by the presence of obesity in this anonymous individuals group.

For each status, overall significance was assessed using the Fisher's method which combines p-values of each independent analysis. The number of effective tests (Nyholt, 2004) was used at each step, 16.72 and 1.2 respectively, to correct for multiple testing while accounting for the between SNPs' correlation. The meta-analysis combining evidence of association for obesity gave very significant results: p-value=$1.67.10^{-26}$ and p=$1.07.10^{-24}$ for SNP rs1421085 and rs17817449. In order to exclude a potential undetected stratification effect, these 2 SNPs were genotyped in the parents and sibs of both French obese children and class III obese adults. An over-transmission of the SNP rs1421085 (rs17817449 respectively) obesity "at risk" C (respectively G) allele to both obese children and adults was observed (%transmitted=57%, p-value=$1.10^{-4}$ and %transmitted=66%, p-value=0.00045; %transmitted=57%, p-value=$2.5.10^{-4}$ and %transmitted=62%, p-value=0.005, respectively). An additional cohort comprising 154 families, discordant for severe obesity, (with at least one class III obese and one lean sib) of Swedish descent was further analyzed, and it was also observed over-transmission of the same allele to obese offspring (%transmitted=61%, p-value=0.05 for both SNPs). The overall significance of these three combined family based studies is $2.8.10^{-6}$.

Moreover, in founders of French Childhood Obesity families dataset, it was found a very strong association with BMI corrected for age and sex (β=0.19 [0.09-0.29], p=$8.10^{-5}$ and β=0.17 [0.07-0.27], p=$4.10^{-4}$) for both SNPs. All replication results are displayed in Table 5 below and genotype counts are shown in Table 1 above.

**Table 5**

Analyses and effect estimates in the study populations

| A/ Independent Adult case-control obesity studies | | | | | | |
|---|---|---|---|---|---|---|
| Study | SNP | Genotyped | Obese | Controls | fcase/con | Multiplicative model |
| Initial Adult obesity | rs1421085 | 3278 | 867 | 2411 | 0.52/0.41 | OR=1.56 [1.40-1.75] p=$7.6.10^{-16}$ |
| | rs17817449 | 3273 | 873 | 2400 | 0.51/0.40 | OR=1.56 [1.40-1.75] p=$1.44.10^{-15}$ |
| Swiss Adult obesity | rs1421085 | 1018 | 504 | 514 | 0.52/0.46 | OR=1.26 [1.07-1.49] p=0.0032 |
| | rs17817449 | 1035 | 516 | 519 | 0.50/0.47 | OR=1.21 [1.02-1.43] p=0.01* |

(continued)
Analyses and effect estimates in the study populations

| A/ Independent Adult case-control obesity studies | | | | | | |
|---|---|---|---|---|---|---|
| Study | SNP | Genotyped | Obese | Controls | fcase/con | Multiplicative model |
| | Fisher test statistic : -2*Σln(p-values) | | | | rs1421085 | p=3.54. 1 0$^{-15}$ |
| | Fisher test statistic : -2*Σln(p-values) | | | | rs17817449 | p= 1.99.1 0$^{-14}$ |
| B/ Independent Case-control studies on Childhood obesity | | | | | | |
| French Childhood obesity 1 | rs1421085 | 1681 | 702 | 979 | 0.48,0.41 | OR=1.28 [1.11 - 1.47] p=2.10$^{-5*}$ |
| | rs17817449 | 1683 | 693 | 990 | 0.47,0.40 | OR=1.25 [1.09 - 1.44] p=5.10$^{-4*}$ |
| French Childhood Obesity 2 | rs1421085 | 1001 | 482 | 519 | 0.51,0.40 | OR=1.47 [1.23-1.75] p=1.17.10$^{-5*}$ |
| | rs17817449 | 1010 | 485 | 525 | 0.51,0.40 | OR=1.52 [1.28-1.81] p=1.82.10$^{-6*}$ |
| German Childhood Obesity | rs1421085 | 982 | 283 | 699 | 0.53, 0.40 | OR=1.69 [1.38-2.06] p=3.46.10$^{-7*}$ |
| | rs17817449 | 972 | 281 | 691 | 0.54,0.42 | OR=1.65 [1.35-2.01] p=1.23.10$^{-6*}$ |
| | Fisher test statistic : -2*Σln(p-values) | | | | rs1421085 | p=9.8.10$^{-14}$ |
| | Fisher test statistic : -2*Σln(p-values) | | | | rs17817449 | p= 1. 7.10$^{-12}$ |
| C/ Overall significance of the case-control studies | | | | | | |
| | Fisher test statistic : -2*Σln(p-values) | | | | rs1421085 | p=1.67.10$^{26}$ |
| | Fisher test statistic : -2*Σln(p-values) | | | | rs17817449 | p=1.07. 10$^{-24}$ |
| D/ Family-based studies | | | | | | |
| Study | | Informative Meïoses | Tr | Non Tr | Tr./Non Tr. | Allelic Model |
| French Childhood Obesity | rs1421085 | 685 | 392 | 293 | 1.37 | p=1.10$^{-4*}$ |
| | rs 17817449 | 707 | 401 | 306 | 1.31 | p=2.5.104' |
| French Adult obesity | rs1421085 | 81 | 73 | 38 | 1.9 | p=0.00045 |
| | rs17817449 | 81 | 70 | 43 | 1.6 | p=0.005* |
| Swedish Adult obesity | rs1421085 | 54 | 33 | 21 | 1.57 | p=0.05* |
| | rs 17817449 | 47 | 29 | 18 | 1.61 | p=0.05* |

(continued)

D/ Family-based studies

| Study | | Informative Meïoses | Tr | Non Tr | Tr./Non Tr. | Allelic Model |
|---|---|---|---|---|---|---|
| Fisher test statistic : -2*Σln(p-values) | | | | | | p=2.8.10$^{-b}$ |

The results of case-control and family-based and analyses are shown in sections A, B and D of Table 5 above, for each cohort. Association analyses compared genotype frequencies in obese and non obese individuals using logistic regression. The OR is the risk increase according to the number of at-risk alleles.

In section C, the significance of the meta-analysis combining all case-controls studies, adults and children is shown. Each p-value is corrected by the number of effective tests inferred from the LD matrix before being added into the Fisher test statistic.

Section D shows the number of transmitted (Tr.) and un-transmitted (Non Tr.) alleles in the three familial samples.

*All the p-values, except those of the initial samples are one-sided.

** Includes trios of grand-parents and parents of the initial childhood obesity study.

### II.2: Results of type II diabetes studies

Table 6 below shows the results of case control analysis on 2400 controls (part of the controls used in the obesity studies described above) and 2200 type II diabetes patients of French Caucasian origin. Analysis was performed under the additive model.

**Table 6**

| Association with Type II diabetes in the *FTO* region | |
|---|---|
| SNP name | p-value additive |
| rs1075440 | 0.000509958 |
| rs7186521 | 0.000594697 |
| rs13334933 | 0.604536 |
| rs16952517 | 0.00221782 |
| rs6499643 | 0.245951 |
| rs4784323 | 0.207249 |
| rs7206790 | 5.67355 10$^{-5}$ |
| rs8047395 | 0.000100524 |
| rs9940128 | 2.37984.10$^{-6}$ |
| rs1421085 | 8.47986.1 0$^{-6}$ |
| rs16952520 | 0.0216816 |
| rs10852521 | 0.00112251 |
| rs1477196 | 0.00301224 |
| rs 1121980 | 3.89511.10$^{-6}$ |
| rs16945088 | 0.0296665 |
| rs17817449 | 2.7426 1.10$^{-5}$ |
| rs8063946 | 0.109686 |
| rs4783819 | 0.00446752 |
| rs3751812 | 6.25013.1 0$^{-5}$ |
| rs11075990 | 2.35022.10$^{-5}$ |
| rs9941349 | 5.16178.10$^{-5}$ |
| rs6499646 | 0.00346673 |
| rs17218700 | 0.816384 |

### III. Conclusions:

*Fatso (FTO)* function is mostly unknown. Mice heterozygous for an *FTO* syntenic Fused toes (Ft) are characterized by partial syndactyly of forelimbs and massive thymic hyperplasia indicating that programmed cell death is affected. Homozygous *Ft/Ft* embryos die at mid-gestation and show severe malformations of craniofacial structures. However, this physical inactivation involves several genes in the region and thus these phenotypes are not necessarily related to *FTO* itself. In humans, a small chromosomal duplication has been identified on large chromosomal 16q12.2 region which includes the *fatso (FTO)* locus (Stratakis *et al.,* 2000). Besides mental retardation, dysmorphic facies, and digital anomalies, the authors also report obesity as primary symptom. *Fatso* (*FTO*) locus variation was also recently reported to be modestly associated with the metabolic syndrome in French Canadian hypertensive families (Seda *et al.,* 2005).

*FTO's* gene expression was examined in several human tissues, especially those of interest for obesity such as brain, adipose tissue, and it was found that human *fatso* gene was expressed in all eleven tested tissues as shown in figure 2. In addition, the microarray based Gene Expression Database of the Novartis Research Foundation's Genomics Institute ("GNF"/SymAtlas) indicates that *fatso* is highly expressed in human hypothalamus, pituitary and adrenal glands suggesting a potential role in the hypothalamic-pituitary-adrenal axis (HPA) implicated in body weight regulation (Su et al., 2004) (http://symatias.gnf.org/SymAtias/). Moreover, the protein has no identified structural domain (Peters *et al.,* 1999) and no informed network link to any other proteins (*Ingenuity* software tools) which could help to predict its function and its physiological role.

Here, it is shown that several potentially functional SNPs in *fatso (FTO)* locus are highly associated with early onset and severe obesity in European population. The calculated Population Attributable Risk of 0.22, which is explained by the high frequency of the at-risk haplotype, argues for a putative important effect on population corpulence. It appears to be the most significant association reported so far for obesity (Lyon *et al.,* 2007). Also, it is shown here that the same SNPs are highly associated with type II diabetes.

It was recently shown that, although most research findings in genetic studies may be accidental, multiple replication of strong associations greatly enhances the positive predictive value of research findings being true, even if the pre study odd is low (Moonesinghe *et al.,* 2007). In this regard, *fatso* appears to be a gene with a strong contribution to obesity as well as type II diabetes despite its, yet, unknown role in glucose homeostasis.

## REFERENCES

1. Peters, T., Ausmeier, K. & Ruther, U. Cloning of Fatso (Fto), a novel gene deleted by the Fused toes (Ft) mouse mutation. Mamm Genome 10, 983-6 (1999).
2. Meyre, D. et al. Variants of ENPP1 are associated with childhood and adult obesity and increase the risk of glucose intolerance and type 2 diabetes. Nat Genet 37, 863-7 (2005).
3. Siepel, A. et al. Evolutionarily conserved elements in vertebrate, insect, worm, and yeast genomes. Genome Res 15, 1034-50 (2005).
4. King, D.C. et al. Evaluation of regulatory potential and conservation scores for detecting cis-regulatory modules in aligned mammalian genome sequences. Genome Res 15, 1051-60 (2005).
5. Ott, J. Association of genetic loci: Replication or not, that is the question. Neurology 63, 955-8 (2004).
6. Hercberg, S. et al. A primary prevention trial using nutritional doses of antioxidant vitamins and minerals in cardiovascular diseases and cancers in a general population: the SU.VI.MAX study--design, methods, and participant characteristics. SUpplementation en VItamines et Mineraux AntioXydants. Control Clin Trials 19, 336-51 (1998).
7. Vu-Hong, T.A. et al. The INS VNTR locus does not associate with smallness for gestational age (SGA) but interacts with SGA to increase insulin resistance in young adults. J Clin Endocrinol Metab 91, 2437-40 (2006).
8. Le Fur, S., Le Stunff, C. & Bougneres, P. Increased Insulin Resistance in Obese Children Who Have Both 972 IRS-1 and 1057 IRS-2 Polymorphisms. Diabetes 51, S304-307 (2002).
9. Korner, A., Berndt, J., Stumvoll, M., Kiess, W. & Kovacs, P. TCF7L2-gene polymorphisms confer an increased risk for early impairment of glucose metabolism and increased height in obese children. J Clin Endocrinol Metab (2007).
10. Nyholt, D.R. A simple correction for multiple testing for single-nucleotide polymorphisms in linkage disequilibrium with each other. Am J Hum Genet 74, 765-9 (2004).
11. Stratakis, C.A. et al. Anisomastia associated with interstitial duplication of chromosome 16, mental retardation, obesity, dysmorphic facies, and digital anomalies: molecular mapping of a new syndrome by fluorescent in situ hybridization and microsatellites to 16q13 (D16S419-D16S503). J Clin Endocrinol Metab 85, 3396-401 (2000).
12. Seda O, T.J., Merlo E, Gaudet D, Broeckel U, Bouchard G, Antoniol G, Brunelle P-L, Gurau A, Gossard F, Pintos J, Kotchen TA, Cowley AW, Hamet P. The Genomic Signatures of Hypertension. Hypertension 46, 875-887 (2005).
13. Lyon, H.N. et al. The association of a SNP upstream of INSIG2 with Body Mass Index is reproduced in several but not all cohorts. PLoS Genetics preprint, e61.eor (2007).
14. Moonesinghe, R., Khoury, M.J. & Janssens, A.C. Most published research findings are false-but a little replication goes a long way. PLoS Med 4, e28 (2007).
15. Su et al. Proc Natl Acad Sci USA 101, 6062-7 (2004).

**Claims**

1. An *in vitro* method for risk assessment and/or diagnosis and/or prognosis of obesity and/or type II diabetes in a human subject, comprising at least:

a) detecting, in a nucleic acid sample from said human subject, at least one biomarker associated with the *FTO* gene; and

b) comparing the biomarker data obtained in step a) from said human subject to biomarker data from healthy and/or diseased people to make risk assessment and/or diagnosis and/or prognosis of obesity and/or type II diabetes in said human subject.

2. The method according to claim 1, wherein said at least one biomarker is selected from the group of single nucleotide polymorphisms (SNPs) listed in Table 2.

3. The method according to claim 1, wherein said at least one biomarker is a polymorphic site associated with at least one SNP selected from the group listed in Table 2.

4. The method according to claim 1, wherein said at least one biomarker is a polymorphic site being in complete linkage disequilibrium with at least one SNP selected from the group listed in Table 2.

5. The method according to anyone of claims 1 to 4, wherein said method is for identifying human subjects at risk for developing obesity and/or type II diabetes.

6. The method according to anyone of claims 1 to 4, wherein said method is for diagnosing obesity and/or type II diabetes in a human subject.

7. The method according to anyone of claims 1 to 4, wherein said method is for selecting efficient and safe therapy to a human subject having obesity and/or type II diabetes.

8. The method according to anyone of claims 1 to 4, wherein said method is for monitoring the effect of a therapy administered to a human subject having obesity and/or type II diabetes.

9. The method according to anyone of claims 1 to 4, wherein said method is for predicting the effectiveness of a therapy to treat obesity and/or type II diabetes in a human subject in need of such treatment.

10. The method according to anyone of claims 1 to 4, wherein said method is for selecting efficient and safe preventive therapy to a human subject at risk for developing obesity and/or type II diabetes.

11. The method according to anyone of claims 1 to 4, wherein said method is for monitoring the effect of a preventive therapy administered to a human subject at risk for developing obesity and/or type II diabetes.

12. The method according to anyone of claims 1 to 4, wherein said method is for predicting the effectiveness of a therapy to prevent obesity and/or type II diabetes in a human subject at risk.

13. The method according to anyone of claims 1 to 12, wherein said at least one SNP listed in Table 2 is selected from rs9940128, rs1421085, rs1121980, rs17817449, rs3751812, rs11075990, and rs9941349.

14. The method according to claim 13, wherein said at least one SNP listed in Table 2 is selected from rs9940128, rs1421085, rs1121980, and rs17817449.

15. The method according to claim 14, wherein said at least one SNP listed in Table 2 is rs1421085 or rs17817449.

16. A test kit for using in an *in vitro* method according to anyone of claims 1 to 15, comprising appropriate means for:

a) assessing type and/or level of at least one biomarker associated with the *FTO* gene in a nucleic acid sample from a human subject; and

b) comparing the biomarker data assessed in a) from said human subject to biomarker data from healthy and/or diseased people to make risk assessment and/or diagnosis and/or prognosis of obesity and/or of type II diabetes in said human subject.

A)

B)

significance in the FTO region (52,34 Mb to 52,40 Mb)

Figure 1

Figure 2

**European Patent Office**

## PARTIAL EUROPEAN SEARCH REPORT

which under Rule 63 of the European Patent Convention shall be considered, for the purposes of subsequent proceedings, as the European search report

Application Number

EP 07 10 8984

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | DINA CHRISTIAN ET AL: "Variation in FTO contributes to childhood obesity and severe adult obesity." NATURE GENETICS JUN 2007, vol. 39, no. 6, June 2007 (2007-06), pages 724-726, XP002465134 ISSN: 1061-4036 * the whole document * | 1-16 | INV. C12Q1/68 |
| P,X | SCOTT LAURA J ET AL: "A genome-wide association study of type 2 diabetes in Finns detects multiple susceptibility variants." SCIENCE (NEW YORK, N.Y.) 1 JUN 2007, vol. 316, no. 5829, 1 June 2007 (2007-06-01), pages 1341-1345, XP002465135 ISSN: 1095-9203 * the whole document * -/-- | 1-16 | |

| | |
|---|---|
| | TECHNICAL FIELDS SEARCHED (IPC) |
| | C12Q |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 18 January 2008 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**European Patent Office**

**PARTIAL EUROPEAN SEARCH REPORT**

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| P,X | FRAYLING TIMOTHY M ET AL: "A common variant in the FTO gene is associated with body mass index and predisposes to childhood and adult obesity." SCIENCE (NEW YORK, N.Y.) 11 MAY 2007, vol. 316, no. 5826, 11 May 2007 (2007-05-11), pages 889-894, XP002465136 ISSN: 1095-9203 * the whole document * | 1-16 | |
| X | EP 1 736 553 A (CENTRE NAT RECH SCIENT [FR]; UNIV DROIT ET DE LA SANTE DE L [FR]) 27 December 2006 (2006-12-27) * claims 1-3,7,18,28,30 * * paragraphs [0033], [0034] * | 16 | TECHNICAL FIELDS SEARCHED (IPC) |
| A | SLADEK ROBERT ET AL: "A genome-wide association study identifies novel risk loci for type 2 diabetes." NATURE 22 FEB 2007, vol. 445, no. 7130, 22 February 2007 (2007-02-22), pages 881-885, XP002465137 ISSN: 1476-4687 * the whole document * -/-- | | |

European Patent
Office

**PARTIAL EUROPEAN SEARCH REPORT**

Application Number

EP 07 10 8984

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | STRATAKIS C A ET AL: "Anisomastia associated with interstitial duplication of chromosome 16, mental retardation, obesity, dysmorphic facies, and digital anomalies: molecular mapping of a new syndrome by fluorescent in situ hybridization and microsatellites to 16q13 (D16S419-D16S503)." THE JOURNAL OF CLINICAL ENDOCRINOLOGY AND METABOLISM SEP 2000, vol. 85, no. 9, September 2000 (2000-09), pages 3396-3401, XP002465138 ISSN: 0021-972X * the whole document * ----- | | |
| A | CAMPFIEL L A ET AL: "Strategies and Potential Molecular Targets for Obesity Treatment" SCIENCE, AMERICAN ASSOCIATION FOR THE ADVANCEMENT OF SCIENCE,, US, vol. 280, 29 May 1998 (1998-05-29), pages 1383-1387, XP002977798 ISSN: 0036-8075 * the whole document * ----- | | TECHNICAL FIELDS SEARCHED (IPC) |

EPO FORM 1503 03.82 (P04C10)

**European Patent Office**

**INCOMPLETE SEARCH
SHEET C**

Application Number

EP 07 10 8984

Claim(s) searched incompletely:
        1-16

Reason for the limitation of the search:

Present claims 1-16 relate to  biomarkers which have a given desired
property or effect, namely to be associated with the FTO gene. However,
the description does not provide support and disclosure in the sense of
Article 84 and 83 EPC for any such biomarkers having the said property or
effect, except for those SNPs of the FTO gene which are disclosed in
tables 2,3 and 6. This non-compliance with the substantive provisions is
to such an extent, that a meaningful search of the whole claimed
subject-matter of the claims could not be carried out (Rule 63 EPC and
Guidelines B-VIII, 3).

The search of claims 1-16 was consequently restricted to the specific
biomarkers having the desired property or effect , namely the SNPs of the
FTO gene as summarized in tables 2,3 and 6.

## ANNEX TO THE EUROPEAN SEARCH REPORT
## ON EUROPEAN PATENT APPLICATION NO.

EP 07 10 8984

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2008

| Patent document cited in search report | | Publication date | Patent family member(s) | Publication date |
|---|---|---|---|---|
| EP 1736553 | A | 27-12-2006 | WO 2006134154 A2 | 21-12-2006 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PETERS, T. ; AUSMEIER, K. ; RUTHER, U.** Cloning of Fatso (Fto), a novel gene deleted by the Fused toes (Ft) mouse mutation. *Mamm Genome,* 1999, vol. 10, 983-6
- **MEYRE, D. et al.** Variants of ENPP1 are associated with childhood and adult obesity and increase the risk of glucose intolerance and type 2 diabetes. *Nat Genet,* 2005, vol. 37, 863-7
- **SIEPEL, A. et al.** Evolutionarily conserved elements in vertebrate, insect, worm, and yeast genomes. *Genome Res,* 2005, vol. 15, 1034-50
- **KING, D.C. et al.** Evaluation of regulatory potential and conservation scores for detecting cis-regulatory modules in aligned mammalian genome sequences. *Genome Res,* 2005, vol. 15, 1051-60
- **OTT, J.** Association of genetic loci: Replication or not, that is the question. *Neurology,* 2004, vol. 63, 955-8
- **HERCBERG, S. et al.** A primary prevention trial using nutritional doses of antioxidant vitamins and minerals in cardiovascular diseases and cancers in a general population: the SU.VI.MAX study--design, methods, and participant characteristics. SUpplementation en VItamines et Mineraux AntioXydants. *Control Clin Trials,* 1998, vol. 19, 336-51
- **VU-HONG, T.A. et al.** The INS VNTR locus does not associate with smallness for gestational age (SGA) but interacts with SGA to increase insulin resistance in young adults. *J Clin Endocrinol Metab,* 2006, vol. 91, 2437-40
- **LE FUR, S. ; LE STUNFF, C. ; BOUGNERES, P.** Increased Insulin Resistance in Obese Children Who Have Both 972 IRS-1 and 1057 IRS-2 Polymorphisms. *Diabetes,* 2002, vol. 51, 304-307

- **KORNER, A. ; BERNDT, J. ; STUMVOLL, M. ; KIESS, W. ; KOVACS, P.** TCF7L2-gene polymorphisms confer an increased risk for early impairment of glucose metabolism and increased height in obese children. *J Clin Endocrinol Metab,* 2007
- **NYHOLT, D.R.** A simple correction for multiple testing for single-nucleotide polymorphisms in linkage disequilibrium with each other. *Am J Hum Genet,* 2004, vol. 74, 765-9
- **STRATAKIS, C.A. et al.** Anisomastia associated with interstitial duplication of chromosome 16, mental retardation, obesity, dysmorphic facies, and digital anomalies: molecular mapping of a new syndrome by fluorescent in situ hybridization and microsatellites to 16q13 (D16S419-D16S503. *J Clin Endocrinol Metab,* 2000, vol. 85, 3396-401
- **SEDA O, T.J. ; MERLO E ; GAUDET D ; BROECKEL U ; BOUCHARD G ; ANTONIOL G ; BRUNELLE P-L ; GURAU A ; GOSSARD F ; PINTOS J.** The Genomic Signatures of Hypertension. *Hypertension,* 2005, vol. 46, 875-887
- **LYON, H.N. et al.** The association of a SNP upstream of INSIG2 with Body Mass Index is reproduced in several but not all cohorts. *PLoS Genetics preprint,* 2007, vol. e61
- **MOONESINGHE, R. ; KHOURY, M.J. ; JANSSENS, A.C.** Most published research findings are false-but a little replication goes a long way. *PLoS Med,* 2007, vol. 4, e28
- **SU et al.** *Proc Natl Acad Sci USA,* 2004, vol. 101, 6062-7